# EUROPEAN PATENT APPLICATION

(11) **EP 2 338 568 A1**
(43) Date of publication of application: **29.06.2011**
(21) Application number: 08877030.0
(22) Date of filing: 26.09.2008
(51) Int. Cl.: A61N 5/06

(54) **PORTABLE PHYSICAL THERAPY DEVICE FOR FAR-INFRARED RAY DIATHERMAL MOXIBUSTION**

(71) Applicant: Lin, Kevin, Hong Kong (CN)
(72) Inventor: Lin, Kevin, Hong Kong (CN)
(74) Representative: Johansson, Lars E.
(86) International application number: PCT/CN2008/072548
(87) International publication number: WO 2010/034158

(57) **Abstract**

A portable far-infrared physiotherapy equipment for thermal moxibustion may comprise at least a heating unit and a power control device connected to the heating unit for supplying power to the latter. The power control device may comprise: a charging control circuit connected to a power supply port to receive power supply from an external power source; an electricity storage unit connected to the charging control circuit to be charged; a power output control unit connecting the charging control circuit and the electricity storage unit to the heating unit respectively to enable to transfer power to the heating unit; and a micro controller unit connected to the electricity storage unit, the charging control circuit and the power output control unit to enable to control them. The present far-infrared physiotherapy equipment for thermal moxibustion is portable and convenient for users.

## Description

### FIELD OF THE INVENTION

The present invention relates to the field of a physiotherapy equipment, particularly to the field of a portable far-infrared physiotherapy equipment for thermal moxibustion.

### BACKGROUND OF THE INVENTION

Physiotherapy is also called physical therapy, which is used for disease prevention or treatment by applying artificial or natural physical factors on human body to generate a favorable reaction thereto. Common artificial physical factors include electricity, light, sound, magnetism, temperature, mechanical force, etc.

Generally, physiotherapy equipments, such as thermal physiotherapy equipments, using temperature as a physical factor are used widely. However, such traditional thermal physiotherapy equipments usually operate at very high temperature to enable thermal energy generated by the equipments to penetrate skin of a user to reach a target portion in the user. Thereby, the user is not tolerant such a high temperature on one hand, while the target portion could not be heated to reach a desired temperature on the other hand. Thus, the skin of the user is subject to being burnt, while the effect of physical therapy could not be achieved, and thereby it will get half the results with twice the effort.

Currently, there is a kind of far-infrared physiotherapy equipment without heating function in the market, which is a passive far-infrared physiotherapy equipment. However, such far-infrared physiotherapy equipment could not generate far-infrared radiations with thermal energy, and thus the emissivity of far-infrared radiations thereof is very low, and the effect of disease prevention or treatment could not be achieved.

Although an active far-infrared physiotherapy equipment could generate far-infrared radiations with thermal energy, such equipment would consume a lot of electric energy, and should be connected to a fixed power source. Therefore, such equipment should be used by users at certain locations and is not portable.

### SUMMARY OF THE INVENTION

To overcome the above-mentioned defects, the main object of the present invention is to provide a portable far-infrared physiotherapy equipment for thermal moxibustion, which may comprise at least a heating unit and a power control device connected to the heating unit for supplying power to the latter, and the power control device may comprise: a charging control circuit connected to a power supply port to receive power supply from an external power source; an electricity storage unit connected to the charging control circuit to be charged; a power output control unit, which is used to connect the charging control circuit and the electricity storage unit to the heating unit respectively to enable to transfer power from the electricity storage unit to the heating unit or from the external power source to the heating unit via the charging control circuit; and a micro controller unit connected to the electricity storage unit, the charging control circuit and the power output control unit, in which the micro controller unit selectively commands the external power source to supply power to the power output control unit via the charging control circuit or the electricity storage unit to supply power to the power output control unit according to the determination on whether the charging control circuit receives power input from the external power source or not, and controls the operation mode of the heating unit by controlling the power supply mode from the power output control unit to the heating unit.

The power control device may further comprise a temperature sensor which is provided near the heating unit to detect the temperature of the heating unit and is connected to the micro controller unit to transmit signals concerning the detected temperature to the micro controller unit, and the micro controller unit compares the detected temperature to a pre-determined temperature of the heating unit stored in a storage unit. In addition, the power control device may further comprise an operation mode selector connected to the micro controller unit, through which a user selects the operation modes of the hating unit and transmit a corresponding signal to the micro controller unit.

Preferably, the operation modes of the heating unit may comprise a heating-once mode and a continuous-heating mode, when the micro controller unit receives a signal of heating-once mode from the operation mode selector, it commands the power output control unit to supply power to the heating unit until the temperature of heating unit reaches the pre-determined temperature; when the micro controller unit receives a signal of continuous-heating mode from the operation mode selector, it commands the power output control unit to supply power to the heating unit periodically in a series of power supply cycles. Each of the power supply cycle may comprise: a full power supply stage, in which the power output control unit supplies power to the heating unit continuously to make the temperature of the heating unit to approach to the pre-determined temperature rapidly; a temperature rise stage with intermittent power supply, in which the power output control unit supplies power to the heating unit intermittently and the power supply period is longer than the suspension period, to enable to raise the temperature of heating unit to the pre-determined temperature step by step; a maintenance stage with intermittent power supply, in which the power output control unit supplies power to the heating unit intermittently and the power supply period is shorter than the suspension period, to enable to keep the temperature of the heating unit; and a power supply termination stage, in which the power output control unit stops supplying power to the heating unit for a certain period of time.

Preferably, the micro controller unit could set the total period of time of the first three stages as 5-60 minutes, while set the period of time of the power supply termination stage as 0-30 minutes.

Alternatively, the power control device may further comprise an optimum temperature identification unit connected to the micro controller unit, through which a user transmit a temperature identification signal to the micro controller unit, in which when the micro controller unit receives the temperature identification signal, it stores data on the current status of the charging control circuit, the electricity storage unit and the power output control unit to the storage unit as destination data for the control of the micro controller unit to the charging control circuit, the electricity storage unit and the power output control unit.

The heating unit may comprise a substrate, a semiconductor electric heating resistance film coated on the substrate, conduction electrodes provided around the semiconductor electric heating resistance film, and cables connected between the conduction electrodes and the power control device for supplying power to the semiconductor electric heating resistance film. The substrate could be made of ceramics, glass or high temperature resistant plastics. Or, the substrate could be made of stainless steel, aluminum alloy or copper, and an insulating layer is provided between the semiconductor electric heating resistance film and the substrate and between the conduction electrodes and the substrate. The heating unit may further comprise a protection member, and the protection member is provided on a surface of the substrate where the semiconductor electric heating resistance film is coated and is made of ceramics, glass or high temperature resistant plastics.

Preferably, emission enhancement materials for far-infrared radiations are coated on a surface of the substrate opposite to the surface coated with the semiconductor electric heating resistance film. The emission enhancement materials could be selected from aluminum oxide, ferric oxide, silicon oxide, titanium oxide, oxide of rare-earth metal and carbon.

Furthermore, the substrate may be provided with one or more magnets around its periphery.

The portable far-infrared physiotherapy equipment for thermal moxibustion may further comprise a carrier on a surface of which the at least a heating unit is provided. The carrier may be a housing, the heating unit is provided on a surface of the housing, and the housing includes two halves coupling to each other to form an inner cavity in which the power control device is mounted. Or, the carrier may be a housing, and the heating unit is attached to one or both ends of the housing to form an inner cavity in which the power control device is mounted. Or, the carrier may be a wrap, a silicon sheet or a medical plaster, and the heating unit is attached to the warp, the silicon sheet or the medical plaster. The number of the heating unit may be two or more, and the heating unit is fixed to acupuncture points or locations of pain of the user by means of the carrier..

### BRIEF DESCRIPTION OF THE DRAWINGS

The present invention will be described in detail with reference to the accompanying drawings, in which,
Fig. 1 is a schematic view of a first embodiment of a portable far-infrared physiotherapy equipment for thermal moxibustion according to the present invention;
Fig. 2 is a side view of the portable far-infrared physiotherapy equipment for thermal moxibustion of Fig. 1;
Fig. 3 is a block diagram of a first embodiment of a power control device in the portable far-infrared physiotherapy equipment for thermal moxibustion according to the present invention;
Fig. 4 is a block diagram of a second embodiment of the power control device in the portable far-infrared physiotherapy equipment for thermal moxibustion according to the present invention;
Fig. 5 is a schematic view of an embodiment of a heating unit used in the portable far-infrared physiotherapy equipment for thermal moxibustion according to the present invention;
Fig. 6 is a side view of the heating unit of Fig. 5;
Fig. 7 is a schematic view of the heating unit of Fig. 5 with a protection member detached;
Fig. 8 is a schematic view of a modification of the heating unit of Fig. 5 with a protection member detached;
Fig. 9 is a schematic view of another embodiment of the heating unit used in the portable far-infrared physiotherapy equipment for thermal moxibustion according to the present invention;
Fig. 10 is a schematic view of a second embodiment of the portable far-infrared physiotherapy equipment for thermal moxibustion according to the present invention;
Fig. 11 is a schematic view of a third embodiment of the portable far-infrared physiotherapy equipment for thermal moxibustion according to the present invention; and
Fig. 12 is a side view of the portable far-infrared physiotherapy equipment for thermal moxibustion of Fig. 11.

### DETAILED DESCRIPTION OF THE INVENTION

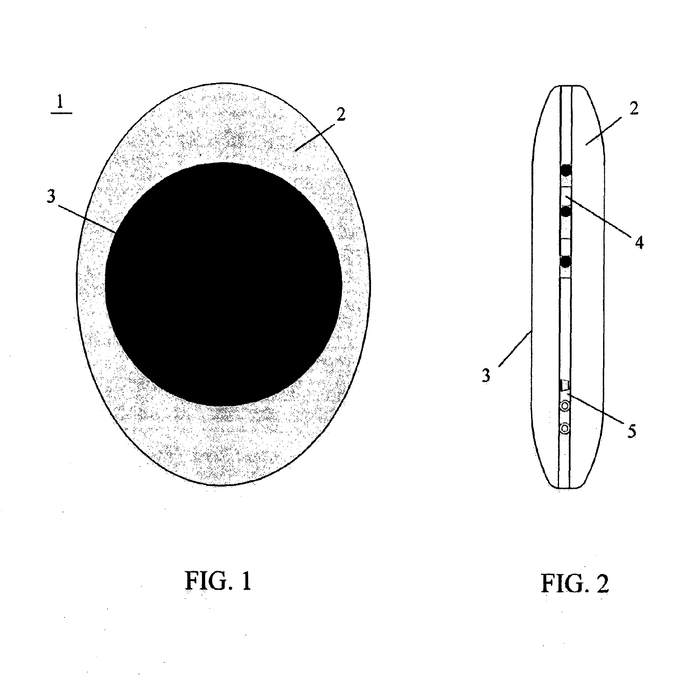

Fig. 1 illustrates the portable far-infrared physiotherapy equipment for thermal moxibustion 1 of the present invention, which comprises a housing 2 and at least a heating unit 3 attached to a surface of the housing. Fig. 2 illustrates that each of the opposite surfaces of the housing 2 is provided with a heating unit 3. However, each surface of the housing could be provided with two or more heating units 3.

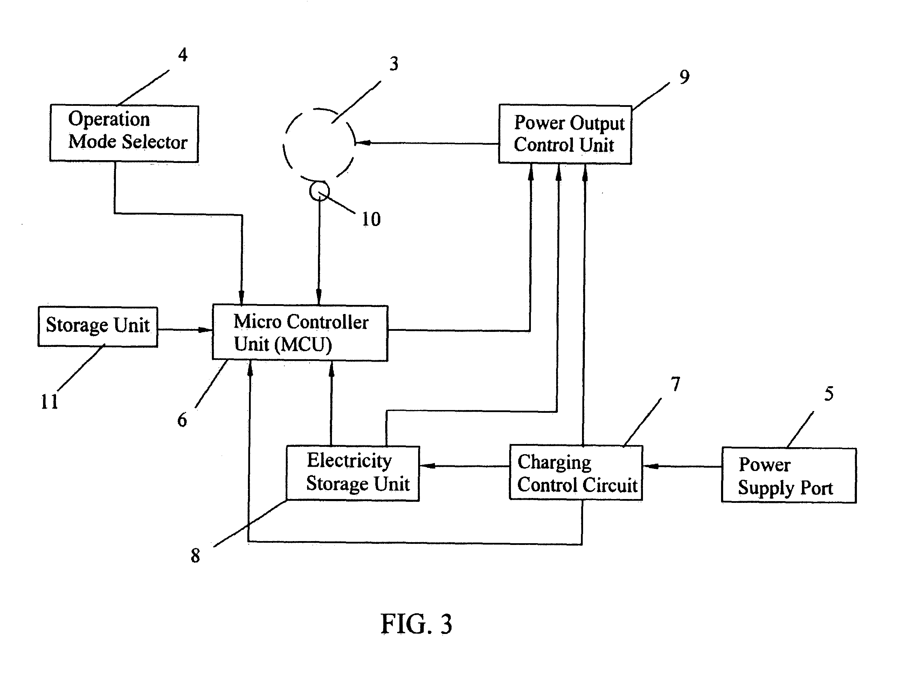

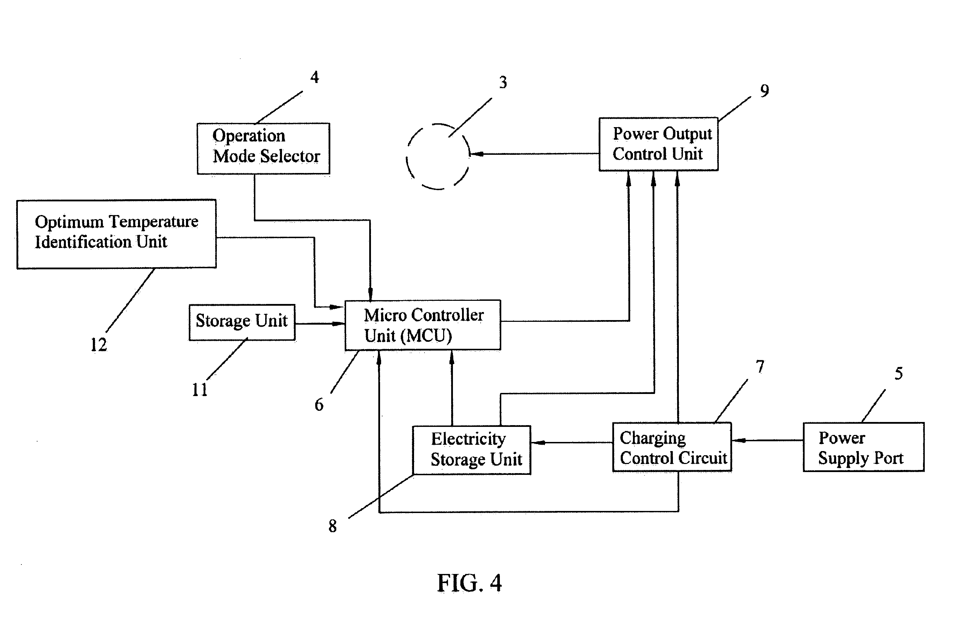

As shown in Fig. 2, the housing 2 may include two halves coupling to each other, to form an inner cavity. The inner cavity of the housing could be used to mount therein a power control device for supplying power to the heating unit 3. The power control device would be described in details with reference to Figs. 3 and 4.

Fig. 3 is a block diagram of a first embodiment of the power control device in the portable far-infrared physiotherapy equipment for thermal moxibustion of the present invention. The power control device may comprise: a micro controller unit (MCU) 6; a charging control circuit 7 connected to a power supply port 5 provided at a side of the housing, for receiving power supplied from an external power source; an electricity storage unit 8 connected to the charging control circuit 7, to enable to be charged; a power output control unit 9 through which the charging control circuit 7 and the electricity storage unit 8 are connected to the heating unit 3 respectively, to enable to supply power from the charging control circuit 7 or the electricity storage unit 8 to the heating unit 3; a temperature sensor 10 provided near the heating unit 3 and connected to the MCU 6, for detecting the temperature of the heating unit 3 and transmitting signals concerning the detected temperature to the MCU 6; a storage unit 11 for storing data and pre-determined temperature value needed for performing control.

The power supply port 5 may be, for example, a universal series bus (USB) port for charging the electricity storage unit by means of a computer; an automobile power port for charging the electricity storage unit by an automobile cigarette lighter; and/or a DC port for charging the electricity storage unit by means of a common transformer.

The electricity storage unit 8 may be a rechargeable battery pack or other suitable electricity storage units. The MCU 6 is connected to the charging control circuit 7, the electricity storage unit 8 and the power output control unit 9, to enable to control them. When the MCU 6 determines that the charging control circuit 7 is receiving power supplied from the external power source and charging the electricity storage unit 8, it would stop power supply from the electricity storage unit 8 to the heating unit 3 via the power output control circuit 9, but command power supply directly from the external power source to the heating unit 3 via the charging control circuit 7 and the power output control unit 9. If the MCU 6 determines that the charging control circuit 7 is not supplied with power, it would command the electricity storage unit 8 to supply power to the heating unit 3 by virtue of the power output control unit 9.

The power control device may further comprise an operation mode selector 4 provided at a side of the housing 2, as shown in Fig. 2. The operation mode selector 4 is connected to the MCU 6, for selecting operation modes of the heating unit 3 by users and then transmitting corresponding signals to the MCU 6. Subsequently, the MCU 6 controls the power output control unit 9 according to the received operation mode signals, to enable the heating unit 3 to work in a corresponding operation mode, for example heating-once mode or continuous-heating mode.

The operation of the power control device shown in Fig. 3 is as follows. When the MCU 6 receives a heating-once mode signal from the operation mode selector 4, it would command the power output control unit 9 to transfer power from the charging control circuit 7 or the electricity storage unit 8 to the heating unit 3, to raise the temperature of the heating unit 3 rapidly. At the same time, the temperature sensor 10 detects the temperature of the heating unit 3, and transmits a temperature signal to the MCU 6. The MCU 6 would then compare the temperature of the heating unit to a pre-determined temperature value stored in the storage unit 11. When it determines that the temperature of heating unit is lower than the pre-determined temperature value, the MCU 6 would command the power output control unit 9 to continue supplying power to the heating unit 3. When it determines that the temperature of the heating unit reaches the pre-determined temperature value, the MCU 6 would command the power output control unit 9 to stop power supply to the heating unit 3.

When the MCU 6 receives a continuous-heating mode signal from the operation mode selector 4, it would command the power output control unit 9 to supply power to the heating unit 3 periodically in a series of power supply cycles. Each power supply cycle could be divided into four stages, i.e. a full power supply stage, a temperature rise stage with intermittent power supply, a maintenance stage with intermittent power supply and a power supply termination stage.

In the full power supply stage, the power output control unit 9 supplies power to the heating unit 3 continuously, to make the temperature of the heating unit 3 to approach to a pre-determined temperature rapidly. For example, when the MCU 6 determines that the temperature of the heating unit 3 detected by the temperature sensor 10 is about 80-90% of the pre-determined temperature, it commands the power output control unit 9 to enter into the second stage, i.e. the temperature rise stage with intermittent power supply. Generally, the full power supply stage will be kept for 1-3 minutes.

In the temperature rise stage with intermittent power supply, the power output control unit 9 would supply power to the heating unit 3 intermittently, in which the power supply period is longer than the suspension period, to enable to raise the temperature of heating unit 3 to the pre-determined temperature step by step. For example, according to the status of the electricity storage unit 8 and/or a desired ramp-up rate of temperature, the power output control unit 9 may supply power intermittently, i.e. suspend power supply for a period of 1 second after each period of power supply for 3-5 seconds. If the temperature of heating unit 3 should be raised rapidly to the pre-determined temperature, the power output control unit 9 would suspend power supply for a period of 1 second after each period of power supply for 5 seconds. However, if the temperature of the heating unit 3 should be raised slowly to the pre-determined temperature, the power output control unit 9 would suspend power supply for a period of 1 second after each period of power supply for 3 seconds. But, the ratio of power supply period to suspension period could be set according to actual situation and demand. When the MCU 6 determines that the temperature of the heating unit 3 detected by the temperature sensor 10 reaches the pre-determined temperature, it would command the power output control unit 9 to enter into the third stage, i.e. the maintenance stage with intermittent power supply.

In the maintenance stage with intermittent power supply, the power output control unit 9 would also supply power to the heating unit 3 intermittently. However, in this stage, the power supply period is shorter than the suspension period, to enable to keep the temperature of the heating unit 3 as long as possible, rather than raise its temperature. For example, according to the status of the electricity storage unit 8 and/or a desired period for keeping the temperature of the heating unit, the power output control unit 9 may supply power intermittently, i.e. suspend power supply for a period of 3-5 seconds after each period of power supply for 1 second. However, the ratio of power supply period to suspension period could be set according to actual situation and demand.

After the first three stages in each cycle are kept for a pre-determined period of time, for example, 5-60 minutes, the MCU 6 would command the power output control unit 9 to enter into the fourth stage, i.e. the power supply termination stage, to release the portion of user being treated. For example, the first three stages are kept for a period of 30 minutes, while the power supply termination stage could be kept for a period of 0-30 minutes. In this stage, the MCU 6 stores the temperatures of the heating unit 3 and data for control in the storage unit 11 to enable to retrieve such temperatures and data in the next power supply cycle. Then, the whole power supply cycle is completed. The next power supply cycle repeats the above stages from a full power supply stage. Therefore, under the control of the MCU 6, the charging control circuit 7 or the electricity storage unit 8 may supply power to the heating unit 3 periodically via the power output control unit 9, and the power supply is intermittent in each cycle, to make the heating unit 3 to operate for a longer period of time with less electric energy.

Furthermore, when the present portable far-infrared physiotherapy equipment for thermal moxibustion 1 includes more than one heating units 3, the operation mode selector 4 may further transmit a selection signal to the MCU 6, to enable the MCU 6 to command the power output control unit 9 to supply power to one or more selected heating units 3. Thus, the selected heating units 3 would start to work, while the other heating units 3 not being selected would not work. The selected heating units 3 could work in the same operation mode, or in different operation modes.

Fig. 4 is a block diagram of a second embodiment of the power control device in the portable far-infrared physiotherapy equipment for thermal moxibustion of the present invention. The second embodiment is similar to the first embodiment except that the temperature sensor 10 of the first embodiment is replaced by an optimum temperature identification unit 12 in the second embodiment. The optimum temperature identification unit 12 is connected to the MCU 6, to enable to transmit an identification signal to the latter. The operation of the optimum temperature identification unit 12 is as follows. A user would determine whether the temperature of the heating unit 3 has reached his desired temperature according to his own feeling. When the use feels that the temperature of heating unit 3 has reached his desired temperature, he could send an identification signal to the MCU 6 by mean of the optimum temperature identification unit 12. Thus, the MCU 6 would store the status of the charging control circuit 7 and/or the electricity storage unit 8 and the status of power supply from the power output control unit 9 to the heating unit 3 into the storage unit 11, as destination data for the control of power supply. The MCU 6 could control the relevant members of the present equipment to approach to such destination data in the future. With the optimum temperature identification unit 12, the user could identify an optimum temperature status of the heating unit at any time according to his own feeling, which could satisfy the requirements of different users.

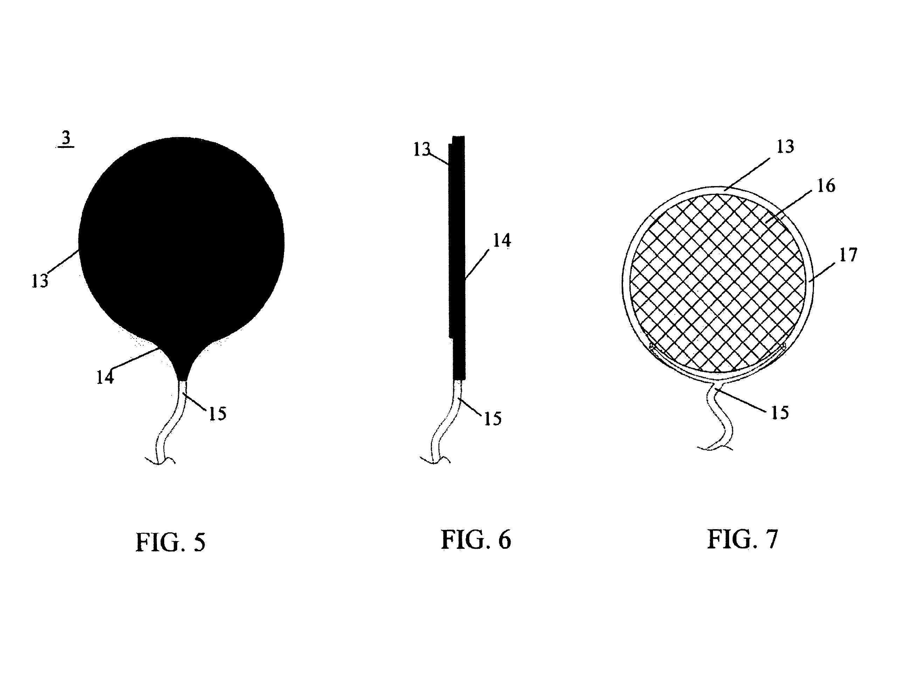

As shown in Fig. 5-7, the heating unit 3 may includes a substrate 13, a semiconductor electric heating resistance film 16 coated on the substrate 13, conduction electrodes 17 provided around the semiconductor electric heating resistance film 16, and cables 15 connected between the conduction electrodes 17 and the above power control device. The substrate 13 may be made of electrical insulating materials such as ceramics, glass or high temperature resistant plastics. Thus, the semiconductor electric heating resistance film 16 and the conduction electrodes 17 could be provided directly on the substrate 13. Or, the substrate 13 could also be made of conductive materials such as stainless steel, aluminum alloy, copper etc. Therefore, before the semiconductor electric heating resistance film 16 and the conduction electrodes 17 are provided on the substrate 13, an insulating layer should be coated on the substrate 13.

Since a power source such as the above power control device supplies power to the semiconductor electric heating resistance film 16 via the cables 15 during operation, the semiconductor electric heating resistance film 16 could be heated and emit far-infrared radiations. After the heating unit 3 is placed and aligned with acupuncture points or locations of pain of users, it could apply far-infrared physical therapy on those points or locations.

To protect the semiconductor electric heating resistance film 16 and the conduction electrodes 17, the heating unit 3 could be further provided with a protection member 14. The protection member 14 is provided on a surface of the substrate 13 where the semiconductor electric heating resistance film 16 is coated. The protection member 14 may be made of insulating materials, such as ceramics, glass or high temperature resistant plastics. The protection member 14 could be used for fixing the cables 15 and thermal insulation. When the heating unit 3 is mounted on the housing 2, the substrate 13 could be faced outwards, and thereby face to the portions of users to be treated.

To improve the emissivity of far-infrared radiations emitted from the heating unit 3, emission enhancement materials for far-infrared radiations, such as aluminum oxide, ferric oxide, silicon oxide, titanium oxide, oxide of rare-earth metal, or carbon etc., could be coated on a surface of the substrate 13 opposite to the surface coated with the semiconductor electric heating resistance film 16.

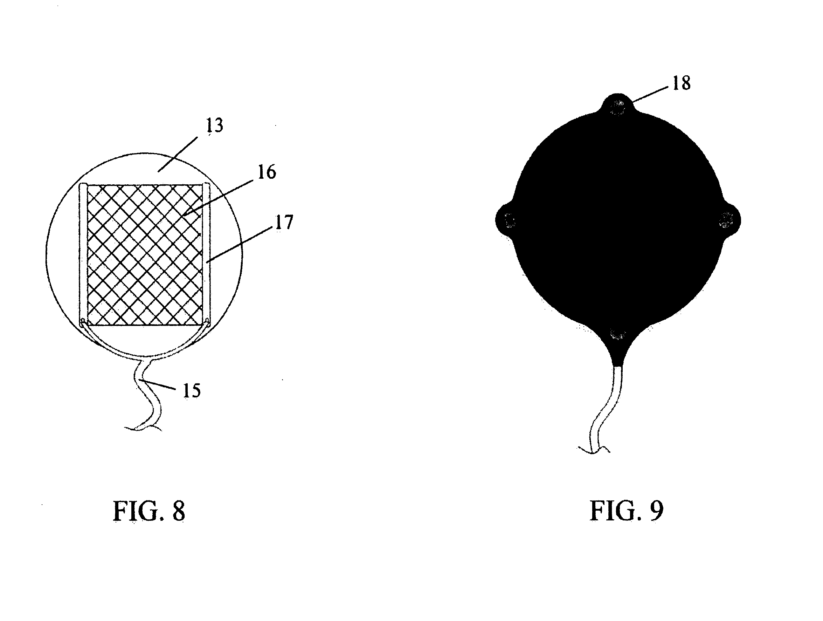

Furthermore, according to different demands, the semiconductor electric heating resistance film 16 could be coated on the substrate 13 in various shapes. For example, Fig. 7 illustrates that the semiconductor electric heating resistance film 16 is in shape of a circle, while Fig. 8 illustrates that the semiconductor electric heating resistance film 16 is in shape of a rectangle.

Fig. 9 illustrates another embodiment of the heating unit according to the present invention. The heating unit shown in Fig. 9 is similar to that shown in Fig. 5 except that the substrate 13 of the heating unit in Fig. 9 is provided with one or more magnets 18 such as permanent magnets around its periphery. Thus, the far-infrared radiations emitted from the heating unit and the magnetic field generated by the magnets could be applied on the portion being treated at the same time.

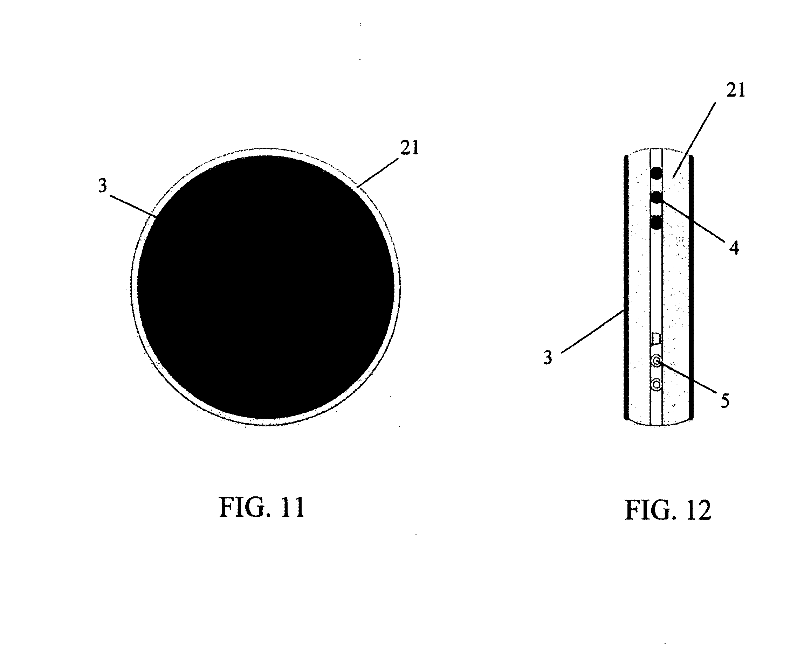

The housing 2 is in shape of an ellipse, as shown in Fig. 1. However, the housing could also be in other shapes. For example, as shown in Figs. 11 and 12, the housing 21 is in shape of a drum. One end or both ends of housing are covered by heating unit(s) 3, to form an inner cavity for containing a power control device. Similarly, an operation mode selector 4 and a power supply port 5 could be provided at a side of the housing. Therefore, the portable far-infrared physiotherapy equipment for thermal moxibustion of the present invention could be used as a massage stone, a sauna stone, a hand warmer, a foot warmer or a body warmer for applying thermal therapy on acupuncture points. After the heating unit 3 provided on the surface of the housing 2 or the heating unit covering the housing 21 is placed and aligned with acupuncture points or locations of pain of users, it could apply far-infrared physical therapy on those points or locations.

However, the heating unit 3 could be formed as a separate member as shown in Figs. 11 and 12, or an external member as shown in Figs. 5-9. During operation, the heating unit 3 is directly connected to a power source such as the above power control device, via the cable 15, and is placed and aligned with acupuncture points or locations of pain of users. Thus, the power control device could supply power to the semiconductor electric heating resistance film 16 of the heating unit 3, to enable the semiconductor electric heating resistance film 16 to generate thermal energy and emit far-infrared radiations, and then apply far-infrared physical therapy on the portion to be treated.

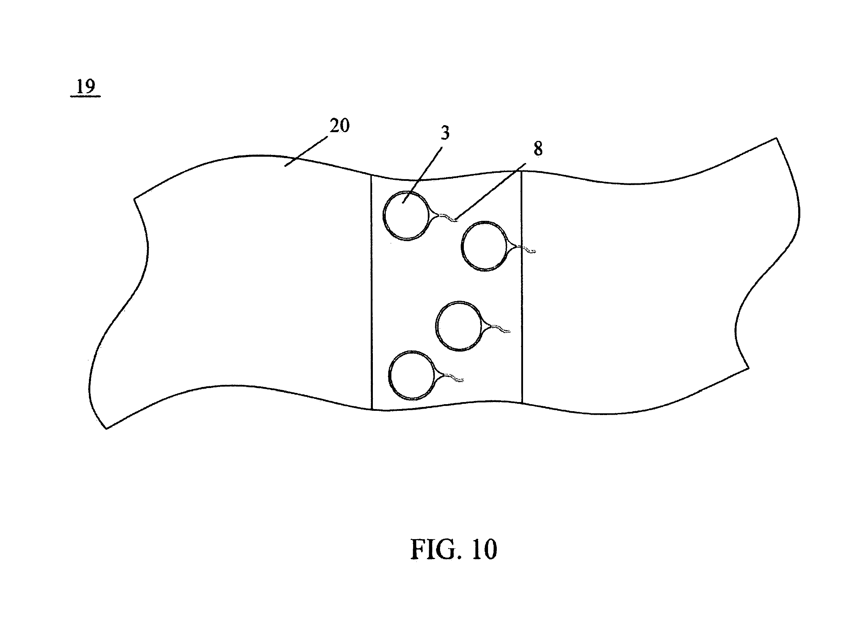

Furthermore, the housing 2 could be formed into various configures according to principle of human engineering, to fit different portions of a user. For example, in order to attach the heating unit 3 to the body or other portions with large size of a user, the above separate or external heating unit could be attached to a wrap 20. Therefore, the physiotherapy equipment of the present invention is formed as a portable far-infrared physiotherapy wrap for thermal moxibustion 19, as shown in Fig. 10. The heating unit 3 is attached to the wrap 20 by virtue of for example, a Velcro, mesh fabric, a binder, etc. Particularly, two or more heating units 3 could be attached to the wrap 20 according to the locations of acupuncture points of human being, and the heating units 3 are connected to the above power control device via cables 15 to be supplied with power by the power control device. After the heating units 3 are placed and aligned with portions to be treated, the wrap 20 could be used to fix the heating units 3. The heating units or external heating units could also be attached to silicon sheets, medical plasters, or other suitable materials for fixing the heating units to human body, and thereby the heating units or external heating units could be placed and aligned to the acupuncture points or locations of pain of users by virtue of the silicon sheets, medical plasters, or the other suitable materials for fixing the heating units.

Semiconductor electric heating resistance films could be used as heating units in the present invention to generate far-infrared radiations, which could emit strong far-infrared radiations at relative low temperature. Thus, the effect of physiotherapy of the present invention is better than other conventional far-infrared physiotherapy equipments. In addition, since the present far-infrared physiotherapy equipment for thermal moxibustion employs semiconductor electric heating resistance films to generate far-infrared radiations, its power consumption is very small. For example, after supplied with power of 0.7W-0.8W under a voltage of 2.5V-3.6V, the heating unit could be heated to 40-44°C, and under the control of the power control device, the heating unit could work in different operation modes. Therefore, the present far-infrared physiotherapy equipment for thermal moxibustion could operate for a longer period of time. Furthermore, the present far-infrared physiotherapy equipment for thermal moxibustion is portable, and is not confined by fixed power sources.

Although the description of the present invention is made with reference to the preferred embodiments, the present invention is not limited to these embodiments. Various modifications and changes can be made to the invention by those skilled in the art without departing from the spirit and scopes of the present invention.

## Claims

1. A portable far-infrared physiotherapy equipment for thermal moxibustion comprising:
at least a heating unit; and
a power control device connected to the heating unit for supplying power to the latter, which comprises:
a charging control circuit connected to a power supply port to receive power supply from an external power source;
an electricity storage unit connected to the charging control circuit to be charged;
a power output control unit, which is used to connect the charging control circuit and the electricity storage unit to the heating unit respectively to enable to transfer power from the electricity storage unit to the heating unit or from the external power source to the heating unit via the charging control circuit; and
a micro controller unit connected to the electricity storage unit, the charging control circuit and the power output control unit, in which the micro controller unit selectively commands the external power source to supply power to the power output control unit via the charging control circuit or the electricity storage unit to supply power to the power output control unit according to the determination on whether the charging control circuit receives power input from the external power source or not, and controls the operation mode of the heating unit by controlling the power supply mode from the power output control unit to the heating unit.

2. The portable far-infrared physiotherapy equipment for thermal moxibustion according to claim 1, wherein the power control device further comprises a temperature sensor which is provided near the heating unit to detect the temperature of the heating unit and is connected to the micro controller unit to transmit signals concerning the detected temperature to the micro controller unit, and the micro controller unit compares the detected temperature to a pre-determined temperature of the heating unit stored in a storage unit.

3. The portable far-infrared physiotherapy equipment for thermal moxibustion according to claim 2, wherein the power control device further comprises an operation mode selector connected to the micro controller unit, through which a user selects the operation modes of the hating unit and transmit a corresponding signal to the micro controller unit.

4. The portable far-infrared physiotherapy equipment for thermal moxibustion according to claim 3, wherein the operation modes of the heating unit comprise a heating-once mode and a continuous-heating mode, when the micro controller unit receives a signal of heating-once mode from the operation mode selector, it commands the power output control unit to supply power to the heating unit until the temperature of heating unit reaches the pre-determined temperature; when the micro controller unit receives a signal of continuous-heating mode from the operation mode selector, it commands the power output control unit to supply power to the heating unit periodically in a series of power supply cycles.

5. The portable far-infrared physiotherapy equipment for thermal moxibustion according to claim 4, wherein each of the power supply cycle comprises: a full power supply stage, in which the power output control unit supplies power to the heating unit continuously to make the temperature of the heating unit to approach to the pre-determined temperature rapidly; a temperature rise stage with intermittent power supply, in which the power output control unit supplies power to the heating unit intermittently and the power supply period is longer than the suspension period, to enable to raise the temperature of heating unit to the pre-determined temperature step by step; a maintenance stage with intermittent power supply, in which the power output control unit supplies power to the heating unit intermittently and the power supply period is shorter than the suspension period, to enable to keep the temperature of the heating unit; and a power supply termination stage, in which the power output control unit stops supplying power to the heating unit for a certain period of time.

6. The portable far-infrared physiotherapy equipment for thermal moxibustion according to claim 5, wherein the micro controller unit sets the total period of time of the first three stages as 5-60 minutes, while sets the period of time of the power supply termination stage as 0-30 minutes.

7. The portable far-infrared physiotherapy equipment for thermal moxibustion according to claim 1, wherein the power control device further comprises an optimum temperature identification unit connected to the micro controller unit, through which a user transmit a temperature identification signal to the micro controller unit, in which when the micro controller unit receives the temperature identification signal, it stores data on the current status of the charging control circuit, the electricity storage unit and the power output control unit to the storage unit as destination data for the control of the micro controller unit to the charging control circuit, the electricity storage unit and the power output control unit.

8. The portable far-infrared physiotherapy equipment for thermal moxibustion according to claim 1, wherein the heating unit comprises a substrate, a semiconductor electric heating resistance film coated on the substrate, conduction electrodes provided around the semiconductor electric heating resistance film, and cables connected between the conduction electrodes and the power control device for supplying power to the semiconductor electric heating resistance film.

9. The portable far-infrared physiotherapy equipment for thermal moxibustion according to claim 8, wherein the substrate is made of ceramics, glass or high temperature resistant plastics.

10. The portable far-infrared physiotherapy equipment for thermal moxibustion according to claim 8, wherein the substrate is made of stainless steel, aluminum alloy or copper, and an insulating layer is provided between the semiconductor electric heating resistance film and the substrate and between the conduction electrodes and the substrate.

11. The portable far-infrared physiotherapy equipment for thermal moxibustion according to claim 8, wherein the heating unit further comprises a protection member, and the protection member is provided on a surface of the substrate where the semiconductor electric heating resistance film is coated and is made of ceramics, glass or high temperature resistant plastics.

12. The portable far-infrared physiotherapy equipment for thermal moxibustion according to claim 8, wherein emission enhancement materials for far-infrared radiations are coated on a surface of the substrate opposite to the surface coated with the semiconductor electric heating resistance film.

13. The portable far-infrared physiotherapy equipment for thermal moxibustion according to claim 12, wherein the emission enhancement materials are selected from aluminum oxide, ferric oxide, silicon oxide, titanium oxide, oxide of rare-earth metal and carbon.

14. The portable far-infrared physiotherapy equipment for thermal moxibustion according to claim 8, wherein the substrate is provided with one or more magnets around its periphery.

15. The portable far-infrared physiotherapy equipment for thermal moxibustion according to any one of claims 8-14 further comprising a carrier on a surface of which the at least a heating unit is provided.

16. The portable far-infrared physiotherapy equipment for thermal moxibustion according to claim 15, wherein the carrier is a housing, the heating unit is provided on a surface of the housing, and the housing includes two halves coupling to each other to form an inner cavity in which the power control device is mounted.

17. The portable far-infrared physiotherapy equipment for thermal moxibustion according to claim 15, wherein the carrier is a housing, and the heating unit is attached to one or both ends of the housing to form an inner cavity in which the power control device is mounted.

18. The portable far-infrared physiotherapy equipment for thermal moxibustion according to claim 15, wherein the carrier is a wrap, a silicon sheet or a medical plaster, and the heating unit is attached to the warp, the silicon sheet or the medical plaster.

19. The portable far-infrared physiotherapy equipment for thermal moxibustion according to claim 18, wherein the number of the heating unit is two or more, and the heating unit is fixed to acupuncture points or locations of pain of the user by means of the carrier.
